(19) Europäisches Patentamt
European Patent Office
Office européen des brevets

(11) Publication number: **0 262 672 B1**

(12) **EUROPEAN PATENT SPECIFICATION**

(45) Date of publication of patent specification: **27.11.91**

(51) Int. Cl.$^5$: **C07C 49/796**, C07C 49/813, C07C 49/84, C07C 225/22, C07C 45/72, G02F 1/35

(21) Application number: **87114326.9**

(22) Date of filing: **30.09.87**

(54) **Non-linear optical article.**

(30) Priority: **30.09.86 JP 229612/86**

(43) Date of publication of application:
**06.04.88 Bulletin 88/14**

(45) Publication of the grant of the patent:
**27.11.91 Bulletin 91/48**

(84) Designated Contracting States:
**CH DE FR GB LI NL SE**

(56) References cited:
**EP-A- 0 021 000**
**EP-A- 0 235 955**

**CHEMICAL ABSTRACTS vol. 95, no. 25, December 21 1981, Columbus, Ohio, USA AL-RAWI, JASSIM et al. "The condensation of chalcones with 1-acetylindole" page 501 * abstract-no. 219 940b ***

(73) Proprietor: **NIPPON OIL AND FATS COMPANY, LIMITED**
**10-1, Yuraku-cho 1-chome**
**Chiyoda-ku Tokyo 100(JP)**

(72) Inventor: **Miyata, Seizo**
**3-18-26, Shimohoya**
**Hoya-shi Tokyo(JP)**
Inventor: **Watanabe, Toshiyuki**
**3-11-13, Honmachi**
**Kurume-sho Tokyo(JP)**
Inventor: **Goto, Yoshitaka**
**2-24-5, Sakuramura Umezono**
**Niihari-gun Ibaraki-ken(JP)**
Inventor: **Nakayama, Masaharu**
**2-15-5, Sakuramura Umezono**
**Niihari-gun Ibaraki-ken(JP)**

(74) Representative: **Wehnert, Werner et al**
**Patentanwälte Dipl.-Ing. Hauck, Dipl.-Phys. Schmitz, Dipl.-Ing. Graalfs, Dipl.-Ing. Wehnert, Dipl.-Phys. Carstens, Dr.-Ing. Döring**
**Mozartstrasse 23**
**W-8000 München 2(DE)**

**EP 0 262 672 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services

CHEMICAL ABSTRACTS, vol. 92, no. 23, June 9, 1980, Columbus, Ohio, USA MITINA V.G. et al. "Kinetic study of the photoismerization of chalcone, its substituted and heterocyclic analogs" page 618 * abstract-no. 197 665z * & Zh. Obshch. Khim. 1980, 50(1), 134-7

CHEMICAL ABSTRACTS, vol. 81, no. 9, September 2, 1974, Columbus, Ohio, USA VASIL'EV R.F. Et al. " Chemiluminescence in the reations of peroxides with highly luminescent amide- substituted oxazole, oxadiazole, and benzoylethylene" page 342

CHEMICAL ABSTRACTS, vol. 74, no.9, March 1, 1971, Columbus, Ohio, USA LAURUSHIN V.F. et al. " Dipole moments and optical characteristics of chalcone derivatives " page 397

CHEMICAL ABSTRACTS,vol. 72, no. 12, March23, 1970, Columbus, Ohio, USA DZURILLA M. et al. " Isothiocyanates and their synthetic precursors VII. Synthesis, infrared and ultraviolet absorption spectra of 4-substituted 3'- and 4' isothiocyanatochalcones" page 353

## Description

The present invention relates to the use of a chemical compound as material for a non-linear optical article or material which exhibits non-linear response when used in an optical instrument or as an optical element or tool.

Non-linear optical materials include those in which non-linear response results due to induced polarization of electrons by the electric field created by the light incident to a material. In other word, the term "non-linear optical material" means the materials for optical uses, which exhibit the so-called non-linear optical effect. In general, such effect is due to the phenomenon which may be indicated by the second and higher order terms in the following equation of:

$$P = \chi^1 E + \chi^2 E \cdot E + \chi^3 E \cdot E \cdot E + \text{------} + \chi^n E \cdot n ;$$

wherein P is polarizability of a material, E is intensity of the electric field, and $\chi^n$ is non-linear sensitivity of the n-th power.

Particularly, when the phenomenon known as the second harmonic generation (SHG), which is obtainable by the utilization of the secondary effect, is intended to apply for optical processing, the incident light is converted into two light waves both having the frequencies corresponding to those of the second harmonic waves. This phenomenon may be conveniently utilized for a variety of optical processing including conversion of wave length, processing of signals and modulation of laser beams.

Crystals of inorganic compounds, such as $KH_2PO_4$ (KDP), $LiNbO_3$, $NH_4H_2PO_4$ (ADP), have hitherto been used as the non-linear optical materials. However, these known materials have several disadvantage, for instance, in that single crystals thereof having high optical purities are very expensive, that they have often deliquescence to absorb moisture in the atmosphere and thus cause problems in handling and that the non-linear sensitivities thereof do not reach a satisfactory level.

On the other hand, since the utility of organic materials for such applications was reported in 1983 in the symposium in the American Chemical Society, there have been some reports which describe the use of crystals of organic compounds as the non-linear optical material. The compounds which have been already reported as the ones having utilities as the non-linear optical materials include, for example, urea, aniline type compounds, and benzalacetophenones including nitro groups.

However, these known organic compounds do not yet exhibit satisfactory non-linear optical effects, or the compounds which exhibit relatively high level non-linear effects have light absorptive terminals that are significantly shifted onto the long wave length range to thus limit the wave length range of the light waves which can be processed therethrough.

Chem. Ab. Vol. 95, No. 25 (1981), pp 501, No. 219940b discloses the condensation of chalcones with l-acetyl indole. However, the literature does not teach that such chalcone derivatives are usable as a non-linear optical material. Chem. Ab. Vol. 92 No. 23 (1980), pp 618, No. 197665Z discloses the photoisomerization of chalcones and derivatives thereof, which, however, fails to touch upon non-linear optical properties of chalcone derivatives. Alternatively, Chem. Ab. Vol. 81 No. 9 (1974), pp 342, No. 49028b describes the reactions of peroxides with amine-substituted oxazole, oxadiazole or benzoylethylene. The literature does not disclose chalcone derivatives nor their non-linear optical properties. Chem. Ab. Vol. 74, No. 9 (1971), pp 397, No. 47614W discloses chalcone derivatives, some of which are the same as those of the present invention. However, in the literature there are no teachings that such compounds have non-linear optical characteristics. Chem. Ab. Vol. 72, No. 12 (1970), pp 353, No. 65551y discloses some chalcone derivatives used in the present invention, which, however, does not suggest that such derivatives exhibit non-linear optical response. EP-A2-0021000 discloses chalcone derivatives, which, however, do not suggest that the derivatives exert second harmonic generation.

Accordingly, it is the object of the invention to provide a material for a non-linear optical article which exhibits especially satisfactory non-linear optical response, and which is free from the aforementioned problems of the known non-linear optical materials.

This object is achieved by the use of a derivative of benzalacetophenone represented by the following general formula (I):

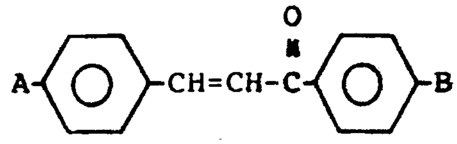

(I)

wherein A and B each represent the same or different atom or group and stand for a hydrogen atom, an alkoxy group having 1 to 4 carbon atoms, a chlorine atom, a bromine atom, an amino group or a dialkylamino group having 1 to 2 carbon atoms.

According to a preferred embodiment of this invention said derivative has a percent transmission of 100% to the visible light rays having the wave lengths of not less than 400 nm.

The single figure appended to this specification is a graph showing the light absorption spectra of example 4 of this invention and a prior art non-linear optical material of comparative Example 1.

The present invention will now be desoribed in detail.

The derivative of benzalacetophenone used in this invention may be prepared by reacting a derivative of benzaldehyde (II) and a derivative of acetophenone (III), the structural formulae (II) and (III) being set forth below, in the presence of a basic or acidic catalyst to effect a dehydrating condensation reaction.

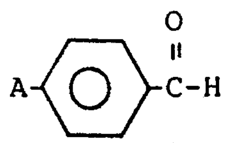

(II)

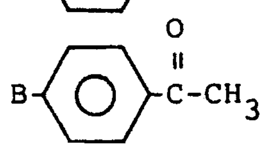

(III)

In the structural formulae (II) and (III), A and B each represent the same or different atom or group and stand for a hydrogen atom, an alkoxy group having 1 to 4 carbon atoms, a chlorine atom, a bromine atom, an amino group or a dialkylamino group having 1 to 2 carbon atoms.

Examples of the basic catalyst which may be used to accelerate the dehydrating condensation reaction are sodium hydroxide, potassium hydroxide, and a variety of quaternary ammonium salts. Examples of the acidic catalyst used for the same purpose are boron trifluoride, phosphorus oxychloride and boron trifluoride etherate.

The derivative of benzalacetophenone used in this invention may be prepared by reacting any one or more of the aforementioned derivatives of benzaldehyde (represented by Formula (II)) and any one or more of the derivatives of acetophenones (represented by the Formula (III)) in the presence of any one or more of the aforementioned catalysts, in an appropriate solvent, such as an alcohol (methanol, ethanol, etc.), at a temperature within the range of from 0°C to 30°C for 30 minutes to 10 hours. Reaction temperatures higher than 30°C are not preferred since various side-reactions take place at such a higher temperature, and reaction temperatures lower than 0°C are also not preferred for economical reasons since the time required for the completion of the reaction is prolonged significantly.

The derivative of benzalacetophenone used according to this invention has an extremely high non-linear optical effect and has a percent transmission of 100% to the visual light rays having wave lengths of not less than 400 nm, and thus it may be used for various optical processings of the light rays as an optical element or tool which is excellent in transparency.

The present invention will now be described more specifically with reference to some examples and comparative examples. However, it should be noted hereby that the following examples are by way of example only but not for limiting sense, and the invention should be limited only by the appended claims.

EXAMPLE 1

Into a reaction vessel provided with a stirrer, 60g of a 10% aqueous sodium hydroxide solution were charged, to which a solution containing 16.2g of paraaminoacetophenone dissolved in 100g of ethanol was added dropwisely, while agitating at 0°C over a period of 15 minutes. After the completion of dropwise addition, a solution containing 16.3g of paramethoxybenzaldehyde dissolved in 50g of ethanol was added

dropwisely at the same temperature over a period of 15 minutes. After the completion of dropwise addition of the paramethoxybenzaldehyde solution, the content in the reaction vessel was raised to 25° C, and then allowed to react for additional 6 hours.

After the completion of the reaction, the separated precipitate was filtered and dried at room temperature for 24 hours under a reduced pressure, whereby 29.5g of a raw product of 3-(4-methoxyphenyl)-1-(4-aminophenyl)-2-propene-1-one was obtained. The yield was 97%.

The raw product was recrystallized from ethanol to obtain 20.7g of a refined product. The yield at this stage was 82%.

The refined product was analysed through a high speed liquid chromatography (using a chromatogram analyser #LC-6A produced by Shimadzu Seisakusho Co. Ltd.) to find that the purity was 99.9%. The melting point of the refined product was 150.5° C.

The second harmonic generation (SHG) was measured in the following manner. A granulated sample having a granule diameter of 50 to 150 microns was sandwiched by slide glass plates, the sample being exposed to a pulse irradiation of an Nd-YAG laser beam (1064 nm) provided with Q-switch for 15 n sec., and second harmonic waves emitted from the sample were detected. Similarly granulated urea was used as the control sample, the relative SHG intensity of the sample was calculated while the SHG intensity of the urea sample was taken as the standard value of 1. This method has been well known to a person having ordinary skill in the art, and disclosed, for example, in Journal of Applied Physics, Vol. 36, No. 8, pages 3798 to 3813 (1968) which will be incorporated herein as a reference.

The results of the determination of the SHG intensity of the sample of this example revealed that the SHG intensity thereof was 30.7 times as high as that generated from urea.

EXAMPLE 2

Into a reaction vessel provided with a stirrer, a solution containing 16.3g of paramethoxybenzaldehyde and 23.9g of parabromoacetophenone dissolved in 120g of dioxane were charged, to which 18g of boron trifluoride etherate was added dropwisely at 0° C over a period of 20 minutes under agitation. After the completion of dropwise addition, the content of the reaction vessel was reacted at 25° C for 7 hours. After the completion of the reaction, the reaction solution was cooled sufficiently with ice, followed by gradual hydrolysis by the addition of water, and then extracted with diethyl ether for three times. The ether phase was rinsed with water and then dried to obtain 37.3g of a raw product of 3-(4-methoxyphenyl)-1-(4-bromophenyl)-2-propene-1-one. The yield of the raw product was 98%.

The raw product was recrystallized from ethanol to obtain 32.2g of a refined prodcut. The yield of the refined product was 85%. The refined product was analysed through a high speed liquid chromatography to find that the purity the reof was 99.7%. The melting point of the refined product was 148.5° C.

Generally following the procedure as described in example 1, the SHG intensity of the refined product was determined to find that the SHG intensity thereof was 13.3 times as high as that of urea.

EXAMPLE 3

Into a reaction vessel provided with a stirrer, 60g of a 10% aqueous solution of sodium hydroxide were charged, to which a solution containing 18g of paramethoxyacetophenone dissolved in 50g of ethanol was added dropwisely at 0° C over 10 minutes under stirring. After the completion of dropwise addition, a solution containing 17.9g of paradimethylaminobenzaldhyde dissolved in 100g of ethanol was added dropwisely at the same temperature over 15 minutes, after which the reaction temperature was raised to 25° C and the reaction was continued for 5 hours.

After the completion of the reaction, the separated precipitate was filtered and then dried at room temperature for 24 hours under a reduced pressure to obtain 32.7g of a raw product of 3-(4-dimethylaminophenyl)-1-(4-methoxyphenyl)-2-propene-1-one. The yield of the raw product was 97%.

The raw product was recrystallized from ethanol to obtain 30.5g of a refined product. The yield of the refined product was 85%.

The refined product was analysed through a liquid chromatography to find that the purity was 99.8%. The melting point of the refined product was 131° C.

The SHG intensity of the sample of this example was determined, similarly as in example 1, to find that the SHG intensity thereof was 20.0 times as high as that of urea.

EXAMPLE 4

Into a reaction vessel provided with a stirrer, a solution containing 22.2g of parabromobenzaldehyde and 18g of paramethoxyacetophenone dissolved in 100g of dioxane was charged, to which there were added dropwisely 17g of boron trifluoride etherate at 0° C over a period of 20 minutes under stirring.

After the completion of dropwise addition, the content in the vessel was reacted at 25° C for 8 hours. After the completion of the reaction, the reaction solution was cooled with ice sufficiently, followed by slow or gentle hydrolysis by the addition of 100g of water, and subjected to extraction with diethyl ether for three times. The ether phase was rinsed with water and then dried to obtain 21.6g of a raw product of 3-(4-bromophenyl)-1-(4-methoxyphenyl)-2-propene-1-one. The yield of the raw product was 98%.

The raw product was recrystallized from ethanol to obtain 18.3g of a refined product. The yield of the refined product was 83%. The refined product was analysed through a high speed liquid chromatography to find that the purity thereof was 99.8%. The melting point of the same was 96° C.

The SHG intensity of the sample of this example was determined, similarly as in example 1, to find that the SHG intensity thereof was 26.7 times as high as that of urea.

EXAMPLE 5

Into a reaction vessel provided with a stirrer, 70g of an 8% aqueous solution of sodium hydroxide were charged, to which there was added dropwisely a solution containing 18g of paramethoxyacetophenone dissolved in 50g of ethanol at 3° C over a period of 10 minutes under stirring. After the completion of dropwise addition, another solution containing 18g of paraethoxybenzaldehyde dissolved in 50g of ethanol was added dropwisely over a period of 10 minutes at the same temperature. After the completion of dropwise addition, the reaction temperature was raised to 25° C, and then the reaction was continued for 6 hours.

After the completion of the reaction, the separated precipitate was filtered and dried at room temperature for 24 hours under a reduced pressure to obtain 32.5g of a raw product of 3-(4-ethoxyphenyl)-1-(4-methoxyphenyl)-2-propene-1-one. The yield of the raw product was 96%.

The raw product was recrystallized from ethanol to obtain 28.8g of a refined product. The yield of the refined product was 85%.

The refined product was analysed through a liquid chromatography to find that the purity thereof was 99.9%. The melting point thereof was 111° C.

The SHG intensity of the sample of this example was determined, similarly as in example 1, to find that the SHG intensity thereof was 20.0 times as high as that of urea.

EXAMPLE 6

Into a reaction vessel provided with a stirrer, 50g of a 10% aqueous solution of sodium hydroxide were charged, to which there was dropwisely added a solution containing 14.4g of acetophenone dissolved in 40g of ethanol at 0° C over a period of 10 minutes while stirring the content in the reaction vessel. After the completion of dropwise addition, a solution containing 22.2g of parabromobenzaldehyde dissolved in 100g of ethanol was added dropwisely at the same temperature over a period of 15 minutes. After the completion of dropwise addition, the reaction temperature was raised to 25° C, and the reaction was continued for 5 hours.

After the completion of the reaction, the separated precipitate was filtered and then dried at room temperature for 24 hours under a reduced pressure to obtain 33.8g of a raw product of 3-(4-bromophenyl)-1-phenyl-2-propene-1-one. The yield of the raw product was 98%.

The raw product was recrystallized from ethanol to obtain 30.3g of a refined product. The yield of the refined product was 88%.

The refined product was analysed through a high speed liquid chromatography to find that the purity thereof was 99.9%. The melting point thereof was 126° C.

The SHG intensity of the sample of this example was determined, similarly as in example 1, to find that the SHG intensity thereof was 13.3 times as high as that of urea.

EXAMPLE 7

The procedure as described in example 2 was repeated except that 14.4g of acetophenone was used in place of parabromoacetophenone as used in example 2, whereby 27g of a raw product of 3-(4-paramethoxyphenyl)-1-phenyl-2-propene-1-one were obtained. The yield was 88%.

The analysis of a refined product through a high speed liquid chromatography revealed that the purity

thereof was 99.9%. The melting point of the same was 76.8° C.

The SHG intensity of the sample of this example was determined, similarly as in example 1, to find that the SHG intensity thereof was 6.7 times as high as that of urea.

COMPARATIVE EXAMPLES 1 to 3

The SHG intensities of the samples of the derivatives of benzalacetophenone having nitro- and methyl-groups, which are described in the article entitled "Organic Materials for Optical Second Harmonic Generation" published in the Collection of Prearranged Report for the Symposium of American Chemical Society held in 1983, were determined in the manners similar to that described in Example 1. The results are set forth in table 1 together with the results of examples 1 to 7.

Table 1

| | Structural Formula | Relative Intensity of SHG |
|---|---|---|
| Example 1 | 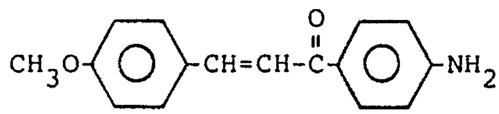 | 30.7 |
| Example 2 | 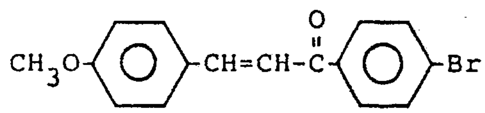 | 13.3 |
| Example 3 | 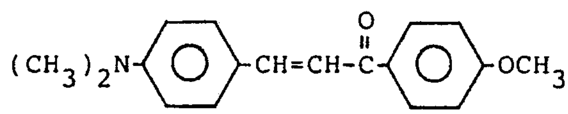 | 20.0 |
| Example 4 | 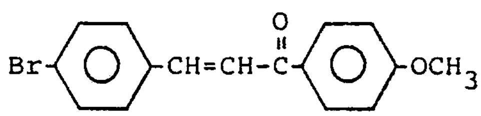 | 26.7 |
| Example 5 | 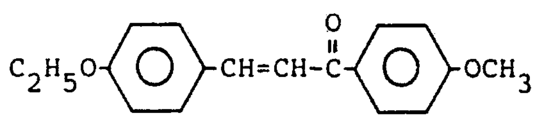 | 20.0 |
| Example 6 | 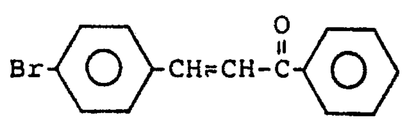 | 13.3 |
| Example 7 | 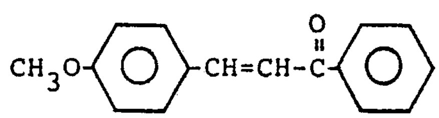 | 6.7 |
| Comparative Example 1 | 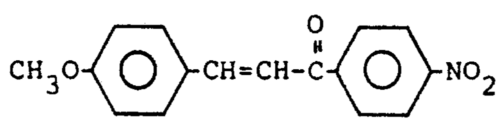 | 5.0 |
| Comparative Example 2 | 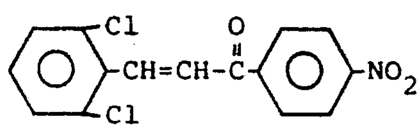 | 4.0 |
| Comparative Example 3 | 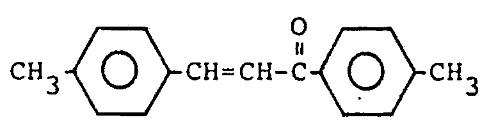 | 1.0 |

## Claims

1. The use of a derivative of benzalacetophenone represented by the following general formula (I) as material for a non-linear optical article:

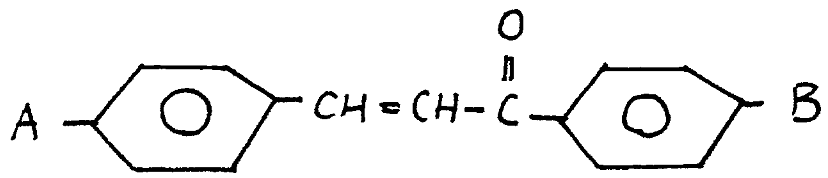

(I)

wherein A and B each represent the same or different atom or group and stand for a hydrogen atom, an alkoxy group having 1 to 4 carbon atoms, a chlorine atom, a bromine atom, an amino group or a dialkylamino group having 1 to 2 carbon atoms.

2. The use according to claim 1 wherein said derivative of benzalacetophenone has a percent transmission of 100 % to the visible light rays having the wave lengths of not less than 400 nm.

**Revendications**

1. L'utilisation d'un dérivé de benzylidèneacétophénone représenté par la formule générale (I) suivante :

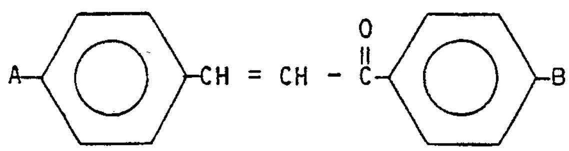

(I)

dans laquelle A et B sont identiques ou différents et représentent chacun un atome d'hydrogène, un groupe alcoxy en $C_1$-$C_4$, un atome de chlore, un atome de brome, un groupe amino ou un groupe dialkylamino ayant 1 ou 2 atomes de carbone comme matériau pour un article optique non linéaire.

2. l'utilisation selon la revendication 1 dans laquelle ledit dérivé de benzylidèneacétophénone a une transmission de 100 % pour la lumière visible ayant des longueurs d'onde de pas moins de 400 nm.

**Patentansprüche**

1. Verwendung eines Derivates von Benzalacetophenon, das durch die nachfolgende allgemeine Formel (I) wiedergegeben wird, als Material für einen nichtlinearen optischen Gegenstand

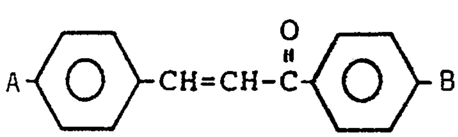

(I)

wobei A und B jeweils das gleiche oder ein unterschiedliches Atom bzw. eine entsprechende Gruppe darstellen und ein Wasserstoffatom, eine Alkoxygruppe mit 1 bis 4 C-Atomen, ein Chlor-Atom, ein Brom-Atom, eine Aminogruppe oder eine Dialkylaminogruppe mit 1 bis 2 C-Atomen bedeuten.

2. Verwendung nach Anspruch 1, wobei das Derivat von Benzalacetophenon einen Durchlässigkeitsgrad von 100 % gegenüber sichtbaren Lichtstrahlen mit Wellenlängen von weniger als 400 nm besitzt.

FIG.

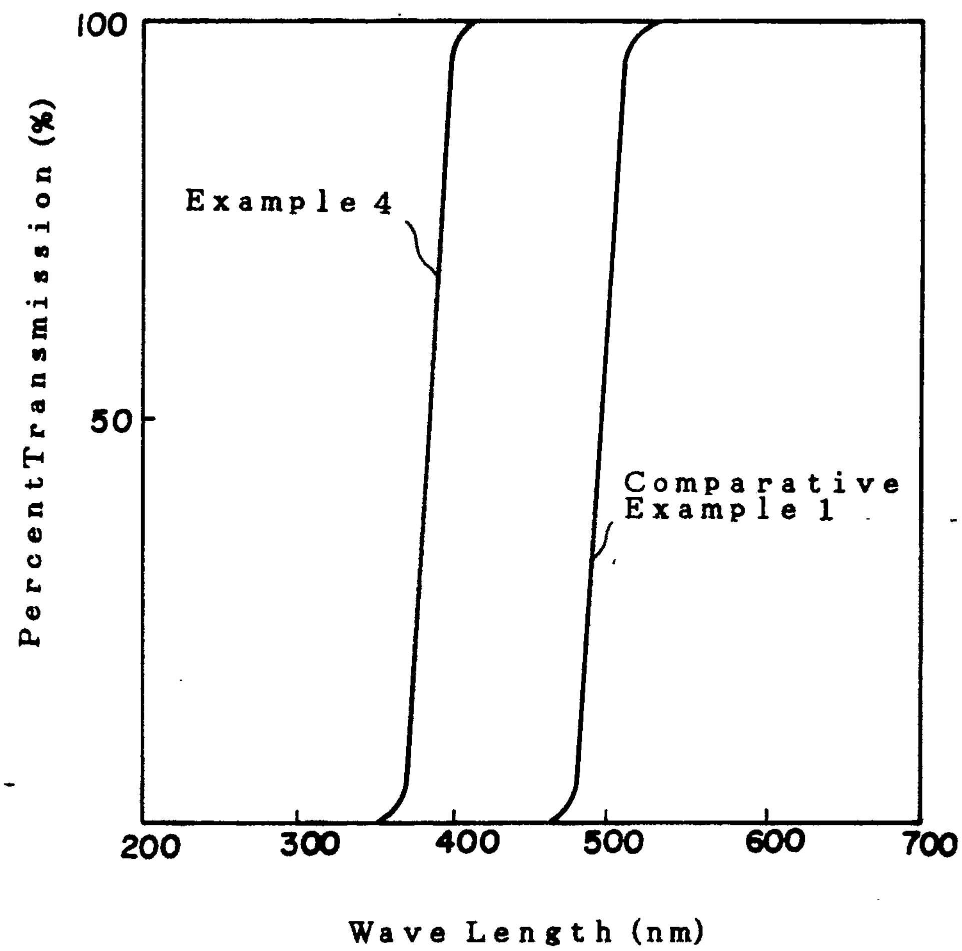
100
50
200
300
400
500
600
700
Percent Transmission (%)
Wave Length (nm)
Example 4
Comparative Example 1